(19) **Europäisches Patentamt / European Patent Office / Office européen des brevets**

(11) **EP 4 461 290 A1**

(12) # EUROPEAN PATENT APPLICATION

(43) Date of publication:
**13.11.2024 Bulletin 2024/46**

(21) Application number: **23173010.2**

(22) Date of filing: **12.05.2023**

(51) International Patent Classification (IPC):
*A61K 8/44* (2006.01)    *A61K 8/60* (2006.01)
*A61Q 11/00* (2006.01)    *A61Q 11/02* (2006.01)
*A61K 31/197* (2006.01)    *A61K 31/7028* (2006.01)
*A61P 1/02* (2006.01)

(52) Cooperative Patent Classification (CPC):
(C-Sets available)
**A61K 8/602; A61K 8/44; A61K 31/197;
A61K 31/7028; A61P 1/02; A61Q 11/00;
A61Q 11/02**    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SkyLab AG**
**1066 Epalinges (CH)**

(72) Inventors:
• **Belous, Elena Yur'evna**
**121309 MOSCOW (RU)**
• **Yarovaya, Alina Olegovna**
**109518 MOSCOW (RU)**

(74) Representative: **Glawe, Delfs, Moll
Partnerschaft mbB von
Patent- und Rechtsanwälten
Postfach 13 03 91
20103 Hamburg (DE)**

(54) **BIO-BASED SURFACTANT COMPOSITION FOR REDUCTION TO REDUCE THE MULTISPECIES BIOFILM FORMATION IN THE ORAL CAVITY**

(57)    The invention relates to a composition comprising rhamnolipid and sodium lauroyl sarcosinate. Said composition na be used as an oral care composition for the prevention or treatment of plaque and or biofilm formation in a mouth and/or teeth.

Fig. 1

EP 4 461 290 A1

(52) Cooperative Patent Classification (CPC): (Cont.)

C-Sets
**A61K 31/197, A61K 2300/00;**
**A61K 31/7028, A61K 2300/00**

**Description**

FIELD OF THE INVENTION

Removal of dental plaque is one of the main problems in oral care faced by the humans. A dental plaque or a dental biofilm is a highly-organized microbial community, which is attached to the solid surface of tooth. On the one hand the biofilm is a commensal part of the immune system, while on the other hand the uncontrolled activity of bacterial pathogens can cause a number of diseases including caries, periodontitis, endodontic infections, tonsillitis, alveolitis, etc. (Yaacob et al., 2014; Colombo et al., 2015; Valm, 2019).

The composition of the dental plaque shows inter-individual variability and depends on a number of factors, however, there are some typical provisions characterizing the dental plaque. The dental plaque is broadly classified as supragingival plaque, which is found at gingival margin or above the gingival margin, and subgingival plaque found below the gingival margin between the tooth and gingival crevice (Haffajee et al., 2008).

There are several stages of the dental biofilm formation: 1) film formation prior to planktonic form attachment, 2) primary colonization followed by proliferation of the attached microorganisms, 3) secondary colonization/coaggregation, 4) biofilm maturation (Souza et al., 2016; Digel et al., 2020). Gram positive cocci and bacilli predominate on the immediate tooth surface, while Gram negative bacilli and filamentous bacteria as well as spirochetes predominate on the surface of the formed biofilm (Haffajee et al., 2008). Besides bacteria the fungi are also involved in the biofilm formation (Valm, 2019).

Mature biofilm is a multilayer structure made of heterogenous cell population surrounded by the extracellular matrix. The nutrients and the bacterial waste products circulate in numerous channels inside the matrix. Matrix is composed of exopolysaccharides, proteins and the macromolecules such as nucleoproteins and lipids. The matrix protects the bacteria from toxins, pH, osmotic pressure changes, ultraviolet radiation and dehydration (Shah et al., 2013).

The shift of equilibrium towards the pathogenic microbes is associated with high risk of development of diseases in oral cavity, such as caries and periodontitis. These are the most common diseases caused by modification of the composition of healthy microbiological society and by entry of the pathogenic microbes from the environment (Tanner et al., 2018; Valm, 2019). In this connection, it is necessary to maintain the healthy oral microbiota and timely removal of cariogenic biofilm. Gingivitis is one more disease, which can be induced by the dental plaque. In the absence of appropriate oral care hygiene, the gingivitis can transform into chronic periodontitis (Kurhan and Kantarci, 2018; Valm, 2019).

In connection with the above timely removal of dental plaque is essential, because uncontrolled dental plaque increase the risk of the oral cavity diseases. At the present time several types of control and prevention of dental plaque are available. These approaches can be classified as mechanical and chemical ones (Vyas et al., 2021). The medical professionals advise to perform the first type of cleaning, mechanical, yearly in the dentist's room (Axelsson and Odont, 1981), where several types of professional care are offered: mechanical treatment, air abrasion, ultrasonic treatment or laser treatment. Between professional cleaning sessions he patient needs to employ prophylactic measures to prevent the dental calculus formation and to decelerate the dental plaque formation. Mechanical methods include the use of toothbrushes, dental flosses, gingival protectors, etc. (Vyas et al., 2021). Toothbrushes are the most commonly used among the above methods (Mandal et al., 2017). For efficacious prophylaxis it is necessary to use safe and efficient chemical agents as the ingredients of prophylactic oral care products.

BACKGROUND OF THE INVENTION

The anti-plaque agents are subdivided into three generations based on various approaches. The first-generation agents, such as antibiotics, phenols and the other compounds, are the compounds decreasing the dental plaque level by 20-50%. The second-generation approach is based on the use of chlorohexidine. Despite efficient (70-90%) removal of dental plaque, this approach has a number of side effects including: hypogeusia, discomfort caused by butter taste, burning sensation, dryness and tenderness of mucosa, and epithelium desquamation. The third-generation agents block the attachment of microbes to the tooth surface (Vyas et al., 2021).

Since many anti-plaque agents are low-efficacious or lead to injury of mucosa, there is unsatisfied need in development of new agents, which would be safe for the soft tissues of oral cavity and efficiently control the plaque formation. This makes very relevant the inclusion of such group of compounds as surfactants into the formulations of oral care products, especially those, which are milder and more environmentally compliant. The authors of the present invention found that composition containing biosurfactant rhamnolipid and sodium lauroyl sarcosinate efficiently prevent the formation of the dental plaque.

Surfactants are chemically surface-active compounds, which are used in the household chemicals, cosmetics, and oral care products. The surfactants are the key component of the toothpastes, where they are responsible for the foaming activity. The surfactants contribute to uniform distribution of the toothpaste in the oral cavity and to the cleaning process, in particular, to removal of debris, microbial biofilm, and the dental plaque from the tooth surface. Another surfactant function is the formation of micelles containing flavor fillers of the toothpaste (Lindenmüller et al., 2011; Aspinall et al., 2021).

Despite beneficial surfactants aspects and the important role thereof in the toothpaste compositions, many synthetic surfactants can case irritation and contamination of the environment subject to entry to the environment (Akbari et al., 2018; Naughton et al., 2019; Aspinall et al., 2021). For instance, large amounts of surfactant can cause the irritation of mucosa, while insufficient amount exhibits considerably lower activity (Aspinall et al., 2021). The surfactants can enter the environment when used in production of various raw materials - in pharmaceutical industry, food and feed industries, agriculture and oil industry.

Safety concerns associated with environmental disadvantages led to establishment of certain standards and social pressure aimed to transition to more biodegradable agents and sustainable production of the raw materials (Naughton et al., 2019). This contributed to reduction of the synthetic surfactant fraction in the toothpastes up to complete absence thereof. However, surfactant-free toothpastes lack useful properties, which characterize the surfactant-based pastes. The solution of choice is to produce the pastes containing biosurfactants or bio-synthetic surfactant compositions to minimize the harmful effects both on oral mucosa and on the environment.

Biosurfactants exhibit a number of advantages compared to synthetic surfactants, such as low toxicity, bioavailability, biodegradation, high foaming activity, environmental friendliness, low cost due to availability of natural raw materials, wide range of pH and salinity, in which the surfactant activity is maintained (Fracchia et al., 2015; Akbari et al., 2021). Besides the use in the oral care products, the biosurfactants are used in the beauty products, pharmaceutics, food industry, agriculture, the textile dyeing. These multifunctional agents exhibit the stabilizing, antimicrobial, moisturizing, emulsifying, and anti-adhesive activities (Banat et al., 2000; Fracchia et al., 2014).

The natural surfactants are produced from the natural raw materials, some of them are produced with the use of microorganisms. The production of biosurfactants exhibiting antibacterial, fungicidal, and antiviral properties by bacteria is of great interest. The bacteria of genera *Pseudomonas* and *Bacillus* have the greatest potential, but taking into account the pathogenicity of these genera the yeast and non-pathogenic bacteria are preferable (Makkar and Cameotra, 2002; Naughton et al., 2019).

The surfactants can be subdivided into the agents produced by chemical synthesis and those obtained from the natural raw materials, i.e, biosurfactants. The latter are classified based on the molecular weight, the origin, and the composition. High-molecular biosurfactants include lipopolysaccharide-protein complexes, polysaccharide-protein-fatty acid or lipoprotein complexes, while glycolipids, lipopeptides, and phospholipids are the low-molecular biosurfactants. Low-molecular biosurfactants more efficiently decrease the surface tension and interfacial tension, while high-molecular biosurfactants are more potent emulsifiers (Banat et al., 2010).

The glycolipids as having the greatest therapeutic potential are of the main interest among the studied biosurfactants. In the glycolipid molecule the carbohydrate fragments are linked to fatty acids of varying length. This group includes trehalolipids, cellobiose lipids, mannosyl erythritol lipids, rhamnolipids, and sophorolipids (Mnif and Ghribi, 2016; Marchant and Banat, 2012; Adu et al., 2020). The glycolipids produced by the microorganisms are exemplified by the rhamnolipids produced by *Pseudomonas spp.,* sophorolipds and mannosyl erythritol lipids produced by *Candida spp.,* trehalose lipids produced by *Mycobacterium* and *Rhodococcus spp* (Santos et al., 2016; Vecino et al., 2017; Peyrat et al., 2019).

Rhamnolipids are mainly synthesized by *Pseudomonas aeruginosa* and the other species of genus *Burkholderia.* The rhamnolipids are classified based on the number of the deoxysaccharide rhamnose residues, which are attached by O-glycoside bond to one or two fatty acid chains. The latter vary in length from 8 to 14 carbon atoms, and 10-carbon rhamnolipids are the most prevalent. One rhamnose gives rise to monorhamnolipid, two rhamnoses give rise to dirhamnolipd (Abdel-Mawgoud et al., 2010; Elshikh et al., 2017).

The rhamnolipids have the greatest potential for expansion at the market of beaty products and household chemicals compared to the other biosurfactants (Müller et al., 2012). This can be explained by the distinguishing properties of rhamnolipids, i.e. the combination of ability to decrease the surface tension, low critical concentration of micelle formation and high washing and foaming activities, antimicrobial properties, gradual substrate sorption, and excellent capacity for molecular complex and liquid crystal formation. Furthermore, the rhamnolipids maintain their properties in the wide temperature, pH, and salinity ranges (Moussa et al., 2014; Sharma, 2016; Kumar and Das, 2018).

Some of synthetic surfactants are non-toxic despite artificial origin and can be used in biodegradable compositions. One of them is sodium lauroyl sarcosinate (SLS). This moderate biodegradable anionic surfactant is made up of a fatty acid and an amino acid sarcosine (Tackie-Otoo et al., 2022). The studies of N-acyl sarcosine derivatives showed that SLS is not a potentially toxic chemical, because the derivatives thereof readily decompose to amino acids and fatty acids (Lanigan, 2001; Tripathy et al., 2018).

The use of rhamnolipids and SLS in the field of oral care products, in particular, for inhibition of the microbial growth, as individual agent and as ingredient of various compositions is reported in the literature.

Elshikh et al. studied the antimicrobial activity of rhamnolipids isolated from non-pathogenic *Burholderia thai-*

*landensis*, against someo microorganisms, which are found in the oral cavity: *Streptococcus mutans, Streptococcus oralis*, *Streptococcus sanguinis, Neisseria mucosa* and *Actinomyces naeslundii.* These microorganisms alongside with some other species are responsible for biofilm formation on the teeth surface, which can lead to dental calculus deposition and caries (Kolenbrander et al., 2010). The authors found that rhamnolipids (both alone and in combination with the other antimicrobial agents, such as tetracycline, chlorhexidine, sodium lauroyl sulphate and ciprofloxacin) prevent the biofilm formation and also destroy existing biofilms (in the absence of any other components). The researchers showed high penetrating capacity of rhamnolipids into the bacterial cells (Elshikh et al., 2017).

*Yamasaki et al.* also demonstrated, in particular, in relation to *A. actinomycetemcomitans Y4, S. mutans* UA159 and *S. sanguinis* ATCC10556, that rhamnolipids inhibit growth and formation of the biofilms in the oral cavity (Yamasaki et al., 2020).

*Mynenivenkatasatya et al.* studied the efficacy of the toothpaste containing SLS and also the stabilized chlorine dioxide and sodium fluoride, in particular, the effect on formation of microbial biofilm. The authors showed high antimicrobial activity of the examiner toothpaste compared to control (Mynenivenkatasatya et al., 2020).

The authors of the present invention showed the unique synergistic action of rhamnolipid when used in combination with SLS. The composition showed high activity against formation of microbial biofilms compared to activities of individual components, i.e., of biosurfactant and SLS. Such unique activity can be associated with the distinguishing properties of individual components, which potentiates due to concomitant use. For instance, SLS, a "green" synthetic surfactant, is also an inhibitor of hexokinase, which is a first enzyme in a metabolic pathway of glucose and some other sugars (Carbon et al., 1995; Fosdick, 1956; Bajani et al., 2018). In this view, the sugars can be metabolized to a lesser extent by bacteria, in particular by *Streptococcus mutans,* thus providing additional teeth protection from the plaque formation and caries development (Forssten et al., 2010). When used with rhamnolipids, which are also efficient surfactants and exhibit antimicrobial activity, in particular, disturb the growth of fungi, bacteria, and other microorganisms, the composition provides and efficacious control over the dental plaque (Abalos et al., 2001; Benincasa et al., 2004; Lourith and Kanlayavattanakul, 2009). Previously the use of rhamnolipids in cosmetics was described, in particular, in the oral care products. Nevertheless, the combination and the unique properties thereof are innovative in nature.

The patent Application US2019307657A1 (EVONIK DEGUSSA GMBH [DE]) published on October 10. 2019, discloses the use of the oral care composition containing at least one biosurfactant and at least one source of fluorine ions. Patented biosurfactants include rhamnolipids, sophorolipids, the lipids containing a glucose residue, trehalolipids, and cellulose lipids. The composition may comprise also the other surfactant of non-biological origin. The authors of the patent highlighted the advantages of the composition, including low level of insoluble fluorine complexes, decreased tooth coloration and deodorization of oral cavity, better taste of the oral care products and better fluorine deposition/retention on the surfaces together with decrease in dental plaque. However, the authors did not demonstrate the decrease in dental plaque weight, therefore this advantage was not technically supported.

The patent Application WO2019133313A1 published on July 4, 2010 (LOCUS IP CO LLC [US]) discloses the use of the composition for amelioration and/or maintenance of the oral health. The composition contains one or more purified biosurfactants and/or derivative thereof, and optionally mat contain one or more carriers, additives and/or adjuvants. The claimed biosurfactants include glycolipids, such as rhamnolipids, sophorolipids, trehalilipids and mannosyl erythritol lipids. While the Applicant indicates the use of the composition as the product for teeth whitening, removal and/or prevention of dental plaque, biofilm or dental calculus and decrease in the population of unfavorable microorganisms in the oral cavity, etc., none of the claimed uses was technically supported. Instead, the authors provided the examples of 1) lipopeptide production by *Bacillus spp.,* 2) fermentation by *Starmerella bombicola* for biosurfactant production, 3) the variant of the tooth paste composition.

The patent Application US2021169757A1 (COLGATE PALMOLIVE CO [US]) discloses the composition for the oral care products, which maintains viscosity, and the methods of use thereof. The ingredients of the composition include 1) basic amino acid, 2) the source of zinc ions, 3) a surfactant system, which comprises one or more alkyl glycosides, acyl glutamates, glycolipids or combinations thereof. According to the authors, the properties of the composition include decrease in acidogenic bacteria population, lesser microbial biofilm formation in the oral cavity, and also decrease or inhibition of the plaque formation. Despite the claimed properties the Applicant demonstrated only the maintenance of viscosity, taste, and foaming activity of the toothpaste. Furthemore, for rhamnolipid-containing pastes only first two characteristics are demonstrated.

The patent US7985722B2 (AURORA ADVANCED BEAUTY LABS [US]) published on July 26, 2011, discloses the use of rhamnolipid-based compositions for cleaning, disinfection, and deodorization of living areas and working rooms. The composition of the invention comprises 0.01% to 99.9% (preferably, 0.01% to 70%) of rhamnolipids, and carrier is the rest. The authors report that such compositions additionally form the biofilm, which prevents proliferation of bacteria and fungi. The patent text alongside with the other numerous optional uses indicates the use of the composition as an ingredient of the toothpastes. However, in the examples of uses pertinent to the oral care products the authors described only the use of the composition for antimicrobial and fungicidal coating on the toothbrushes, where the rhamnolipid antimicrobial properties and the surface tension maintained for a week. Nevertheless, since no experiments demonstrating the claimed rhamnolipid properties of the other oral care products were made, the activity thereof is not proved.

Lauroyl sarcosinate (LS) has been patented as an ingredient of many compositions, though said compositions do not include rhamnolipids. For instance, US patent US2022023180A1 (COLGATE PALMOLIVE CO [US]) published on January 27, 2022, provides the composition containing lauroyl sarcosinate and betaine for use in the oral care products. The authors of the patent described several potential compositions and the foaming activities thereof, however the efficacy against the microbial biofilm was not demonstrated.

Thus, the authors developed the innovative composition containing the rhamnolipids and LS. This composition unexpectedly showed high synergetic efficacy in decrease of the dental plaque weight compared to the individual components and can be used as the ingredient of cosmetic products for efficient cleaning of teeth and oral cavity.

DISCLOSURE OF THE INVENTION

The invention is set out in the appended claims. The invention is related to the composition containing the following two components (A) and (B): (A) rhamnolipid; and (B) sodium lauroyl sarcosinate.

Said rhamnolipid can be produced from genus *Burkholderia* bacteria, preferably, from *Pseudomonas spp.,* more preferably from *Pseudomonas aeruginosa,* and even more preferably said rhamnolipid is identified by CAS 4348-76-9.

The level of component (A) in said composition may be selected from the following ranges, wt %: 0.004-5.000; 0.006-4.000; 0.008-3.000; 0.010-2.000; 0.011-1.900; 0.012-1.800; 0.013-1.700; 0.014-1.600; 0.015-1.500; 0.015-1.400; 0.015-1.300; 0.015-1.200; 0.015-1.100; or 0.015-1.000. For example, the level of component (A) in said composition is selected from the following, wt %: 0.004; 0.005; 0.006; 0.007; 0.008; 0.009; 0.01; 0.015; 0.02; 0.025; 0.03; 0.035; 0.04; 0.045; 0.05; 0.055; 0.06; 0.065; 0.07; 0.075; 0.08; 0.085; 0.09; 0.095; 0.1; 0.15; 0.2; 0.25; 0.3; 0.35; 0.4; 0.45; 0.5; 0.55; 0.6; 0.65; 0.7; 0.75; 0.8; 0.85; 0.9; 0.95; 1; 1.05; 1.1; 1.15; 1.2; 1.25; 1.3; 1.35; 1.4; 1.45; 1.5; 1.55; 1.6; 1.65; 1.7; 1.75; 1.8; 1.85; 1.9; 1.95; 2.00; 2.05; 2.1; 2.15; 2.2; 2.25; 2.3; 2.35; 2.4; 2.45; 2.5; 2.55; 2.6; 2.65; 2.7; 2.75; 2.8; 2.85; 2.9; 2.95; 3.00; 3.05; 3.1; 3.15; 3.2; 3.25; 3.3; 3.,35; 3.4; 3.45; 3.5; 3.55; 3.6; 3.65; 3.7; 3.75; 3.8; 3.85; 3.9; 3.95; 4.00; 4.05; 4.1; 4.15; 4.2; 4.25; 4.3; 4.35; 4.4; 4.45; 4.5; 4.55; 4.6; 4.65; 4.7; 4.75; 4.8; 4.85; 4.9; 4.95; or 5.00. Furthermore, the level of component (A) in said composition may be selected from the following ranges, wt %: 0.06-0.20; 0.07-0.19; 0.08-0.18; 0.09-0.17; 0.1-0.16; 0.11-0.15; 0.12-0.14; or 0.13-0.14. For example, the level of component (A) in said composition is selected from the following, wt %: 0.06; 0.07; 0.08; 0.09; 0.1; 0.11; 0.12; 0.13; 0.14; 0.15; 0.16; 0.17; 0.18; 0.19; or 0.2.

The level of component (B) in said composition may be selected from the following ranges, wt %:: 0.05-22.00; 0.10-11.00; or 0.20-5,50. For example, the level of component (B) in said composition is selected from the following, wt %: 0.05; 0.06; 0.07; 0.08; 0.09; 0.1; 0.15; 0.2; 0.25; 0.3; 0.35; 0.4; 0.45; 0.5; 0.55; 0.6; 0.65; 0.7; 0.75; 0.8; 0.85; 0.9; 0.95; 1; 1.5; 2; 2.5; 3; 3.5; 4; 4.5; 5; 5.5; 6; 6.5; 7; 7.5; 8; 8.5; 9; 9.5; 10; 10.5; 11; 11.5; 12; 12.5; 13; 13.5; 14; 14.5; 15; 15.5; 16; 16.5; 17; 17.5; 18; 18.5; 19; 19.5; 20; 20.5; 21; 21.5; or 22. Furthermore, the level of component (B) in said composition may be selected from the following ranges, wt %:1.0-2.0; 1.1-1.9; 1.2-1.8; 1.3-1.7; 1.4-1.6; or 1.5-1.6. For example, the level of component (B) in said composition is selected from the following, wt %: 1.0; 1.1; 1.2; 1.3; 1.4; 1.5; 1.6; 1.7; 1.8; 1.9; or 2.0.

The weight ratio of sodium lauroyl sarcosinate and rhamnolipid in the combination may be as follows: 0.07:2.0 to 0.004:0.2, respectively. In other words, within said range the weight content can be as follows: 0.004; 0.005; 0.006; 0.007; 0.008; 0.009; 0.01; 0.015; 0.02; 0.025; 0.03; 0.035; 0.04; 0.045; 0.05; 0.055; 0.06; 0.065; 0.07 and 0.2; 0.25; 0.3; 0.35; 0.4; 0.45; 0.5; 0.55; 0.6; 0.65; 0.7; 0.75; 0.8; 0.85; 0.9; 0.95; 1; 1.5; 2 respectively.

Said composition may be an oral care composition.

Said oral care composition may be selected from the group comprising a mouthwash, and toothpaste.

Said composition can be an oral care composition, wherein said oral care composition is preferably a formulation selected from the following: a film, an aerosol, a suspension, a solution, a tincture a cream, a lotion, an ointment, a gel, a powder, or a granulate.

Said composition can be used for nonmedical and/or aesthetic purpose.

In a further aspect the invention is related to the use of composition of the invention for prevention or treatment of forming dental plaque and/or biofilm in the mouth and/or on teeth.

Said dental plaque and/or biofilm may be derived from the oral flora in the mouth, wherein said oral flora can be a microorganism-based oral mouth flora, wherein further preferably said microorganisms may be selected from the group comprising *Streptococcus mutans, Streptococcus oralis, Streptococcus sanguinis, Neisseria mucosa* and *Actinomyces naeslundii.*

In a further aspect the invention is related to a pharmaceutical composition or medicament containing the composition of the invention.

In a further aspect the invention is related to the composition of the invention to be used as a pharmaceutical composition or medicament, wherein said medicament is preferably formulated to be used in the oral cavity of the subject.

Ina further aspect, said medicament or a composition of the invention can be used in treatment of the subject having at least one of the following diseases or symptoms: gingivitis, stomatitis, offensive breath, sensitive teeth, caries or dental calculus.

EXAMPLES

Example 1.

The aqueous solutions of sodium lauroyl sarcosinate, biosurfactant rhamnolipid and the mixture thereof at various concentrations were tested for inhibition of biofilm formation on the surface of rough glass rods. The test sample of the raw material contained sodium lauroyl sarcosinate at the level of 35%. The test sample of the raw material contained rhamnolipid at the level of 40%.

Sterile water was used as a negative control, and the mouthwash Corsodyl, containing 0.2% chlorhexidine was used as a positive control. Dry biofilm mass was weighted to determine the amount of formed dental plaque.

The dental plaque was grown for 3 days by immersion of rough glass rods into the fresh human saliva containing 0.1% saccharose. On Day 2 and Day 3 the growth of biofilm was additionally stimulated by addition of nutritive broth containing tryptone-soya broth (TSB), saliva and saccharose. On Day 1 one treatment of the glass rods with the test component solutions, and on Days 2 and 3 two treatments of the glass rods with the test component solutions were made. At the end of Day 3 the biofilm was collected from the glass rods, and the plaque amount by dry weight (g) was determined. Lesser weight indicated higher efficacy against the dental plaque.

*Experimental procedure:*

Sterilized rods were fixed in the holders in the microbiological cabinet. Since that moment the rods and the holders were used as the integral units.

On Day 1 the sterile glass rods were pre-treated for 2 minutes with the test component solutions taken in the amount of 5 mL. Then the glass rods were removed, washed in 5 mL of the sterile distilled water and immersed into 5 mL of saliva containing 0.1% saccharose. The tubes were placed into the rack and then into the incubator (t=37°C) and on shaker (250 rpm) for about 18 hours.

On Days 2 and 3 in the morning the rods were treated with the test component solutions, then placed into nutritive broth and returned into the incubator and on shaker. After 6 h incubation in the broth the rods were treated with the test component solution once again and transferred into the saliva with 0.1% glucose, and then returned into the incubator for 18 hours.

On Day 4 the tubes with the rods were removed from the incubator and used for determination of the biofilm dry weight. For this purpose, the rods were dried and then weighted on analytical balance with the accuracy of 4 decimal places. The biofilm was removed after the rehydration, and the rods were weighted once again. The biofilm dry weight was determined from the equation:

$$Biofilm\ weight\ (g) = Weight\ of\ the\ road\ covered\ with\ biofilm\ (g) - Rod\ weight\ (g)$$

Experimental results:

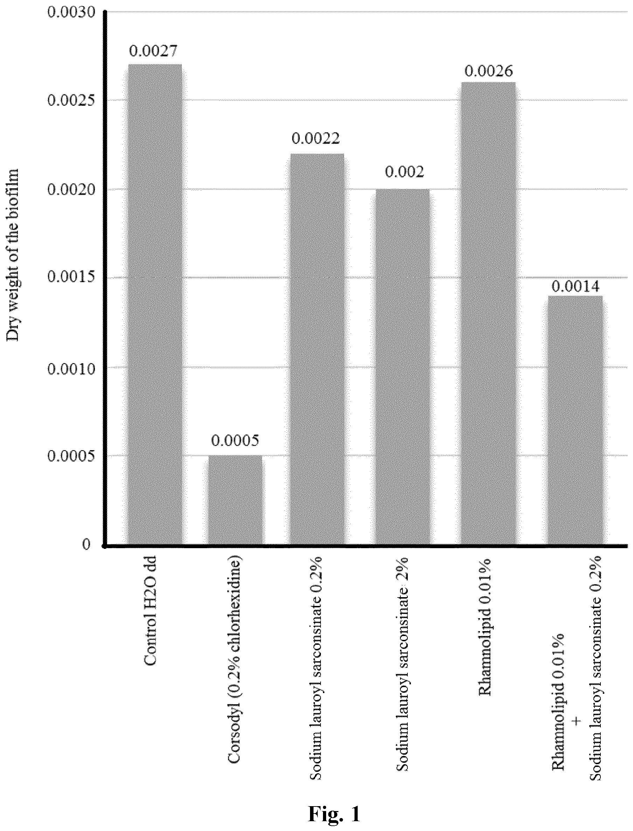

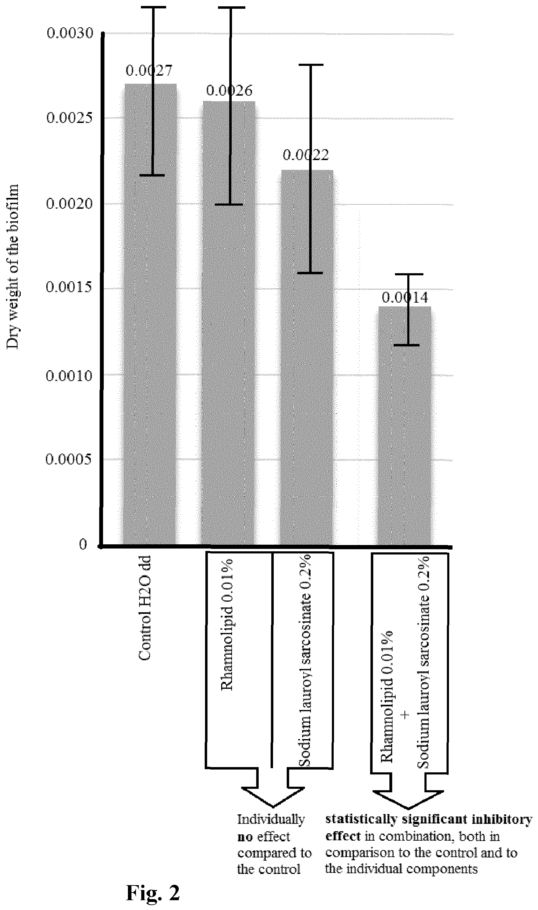

The results obtained for the anti-biofilm activity of the tested components are given in Table 1 and Figure 1, wherein Figure 2 summarizes the core of the invention, i.e. the statistically significant synergistic inhibitory effect of the claimed composition comprising the combination of rhamnolipid and sodium lauroyl sarcosinate compared to the control and said components individually being on the control level.

**Table 1.** Dry weight of biofilm (g).

| Component | Average biofilm weight (g) | Standard deviation |
|---|---|---|
| Biosurfactant rhamnolipid 0.01% | 0.0026 | 0.0006 |
| Sodium lauroyl sarcosinate 0.2% | 0.0022 | 0.0006 |
| Sodium lauroyl sarcosinate 0.2% + Biosurfactant rhamnolipid 0.01% | 0.0014 | 0.0002 |

(continued)

| Component | Average biofilm weight (g) | Standard deviation |
|---|---|---|
| Sodium lauroyl sarcosinate 2% | 0.0020 | 0.0004 |
| Distilled water | 0.0027 | 0.0005 |
| Corsodyl (0.2% chlorhexidine) | 0.0005 | 0.0002 |

As is evident from Table 1, the combination of sodium lauroyl sarcosinate 0.2% and rhamnolipid 0.01% provides significant decrease in the dental plaque formation. The dental plaque weights in the event of the rod treatment with 0.2 and 2% aqueous sodium lauroyl sarcosinate solutions are virtually identical thus indicating the absence of obvious dose-dependent effect: 10-fold increase in concentration of this component does not lead to better efficacy in terms of decrease of the dental plaque formation. However, the addition of just 0.01% rhamnolipid solution to 0.2 sodium lauroyl sarcosinate solution results in considerably lower weight of dental biofilm, thus demonstrating the obvious synergistic effect of the components in tested concentrations.

REFERENCES

1. Abalos A. et al. Physicochemical and antimicrobial properties of new rhamnolipids produced by Pseudomonas aeruginosa AT10 from soybean oil refinery wastes //Langmuir. - 2001. - T. 17. - - № . 5. - C. 1367-1371.

2. Abdel-Mawgoud A. M., Lépine F., Déziel E. Rhamnolipids: diversity of structures, microbial origins and roles //Applied microbiology and biotechnology. - 2010. - T. 86. - № . 5. - C. 1323-1336.

3. Adu S. A. et al. Microbial biosurfactants in cosmetic and personal skincare pharmaceutical formulations //Pharmaceutics. - 2020. - T. 12. - № . 11. - C. 1099.

4. Akbari S. et al. Biosurfactants-a new frontier for social and environmental safety: a mini review //Biotechnology Research and Innovation. - 2018. - T. 2. - № . 1. - C. 81-90.

5. Aspinall S. R., Parker J. K., Khutoryanskiy V. V. Oral care product formulations, properties and challenges //Colloids and Surfaces B: Biointerfaces. - 2021. - T. 200. - C. 111567.

6. Axelsson P., Odont D. Concept and practice of plaque-control //Pediatr Dent. - 1981. - T. 3. - № . Sp. Issue. - C. 101-113.

7. Bajani D., Gharai D., Dey J. A comparison of the self-assembly behaviour of sodium N-lauroyl sarcosinate and sodium N-lauroyl glycinate surfactants in aqueous and aqueo-organic media //Journal of colloid and interface science. - 2018. - T. 529. - C. 314-324.

8. Banat I. M., Makkar R. S., Cameotra S. S. Potential commercial applications of microbial surfactants //Applied microbiology and biotechnology. - 2000. - T. 53. - № . 5. - C. 495-508.

9. Benincasa M. et al. Chemical structure, surface properties and biological activities of the biosurfactant produced by Pseudomonas aeruginosa LBI from soapstock //Antonie Van Leeuwenhoek. - 2004. - T. 85. - № . 1. - C. 1-8.

10. Carbon J. A. et al. The inhibition of anaerobic glycolysis by sodium N-lauroyl sarcosinate //Archives of Biochemistry and Biophysics. - 1955. - T. 55. - - № . 2. - C. 356-364.

11. Elshikh M. et al. Rhamnolipids and lactonic sophorolipids: natural antimicrobial surfactants for oral hygiene //Journal of applied microbiology. - 2017. - T. 123. - № . 5. - C. 1111-1123.

12. Forssten S. D., Björklund M., Ouwehand A. C. Streptococcus mutans, caries and simulation models //Nutrients. - 2010. - T. 2. - № . 3. - C. 290-298.

13. Fosdick L. S. Clinical experiment on the use of sodium N-lauroyl sarcosinate in the control of dental caries //Science. - 1956. - T. 123. - № . 3205. - C. 988-989.

14. Fracchia L. et al. Industrial applications of biosurfactants //Biosurfactants: production and utilization-processes, technologies, and economics. - 2014. - C. 245-260.

15. Fracchia L. et al. Potential therapeutic applications of microbial surface-active compounds //AIMS Bioengineering. - 2015. - T. 2. - № . 3. - C. 144-162.

16. Kolenbrander P. E. et al. Oral multispecies biofilm development and the key role of cell-cell distance //Nature Reviews Microbiology. - 2010. - T. 8. - № . 7. - C. 471-480.

17. Kumar R., Das A. J. Future Prospects and Scenario of Rhamnolipids //Rhamnolipid Biosurfactant. - Springer, Singapore, 2018. - C. 137-141.

18. Kumar R., Das A. J. Industrial Applications of Rhamnolipid: An Innovative Green Technology for Industry //Rhamnolipid Biosurfactant. - Springer, Singapore, 2018. - C. 65-77.

19. Lanigan R. S. Final report on the safety assessment of Cocoyl Sarcosine, Lauroyl Sarcosine, Myristoyl Sarcosine, Oleoyl Sarcosine, Stearoyl Sarcosine, Sodium Cocoyl Sarcosinate, Sodium Lauroyl Sarcosinate, Sodium Myristoyl Sarcosinate, Ammonium Cocoyl Sarcosinate, and Ammonium Lauroyl Sarcosinate //International journal of toxicology. - 2001. - T. 20. - C. 1-14.

20. Lindenmüller I. H., Lambrecht J. T. Oral care //Topical applications and the mucosa. - 2011. - T. 40. - C. 107-115.

21. Lourith N., Kanlayavattanakul M. Natural surfactants used in cosmetics: glycolipids //International journal of cosmetic science. - 2009. - T. 31. - № . 4. - C. 255-261.

22. Makkar R., Cameotra S. An update on the use of unconventional substrates for biosurfactant production and their new applications //Applied microbiology and biotechnology. - 2002. - T. 58. - № . 4. - C. 428-434.

23. Mandal A. et al. New dimensions in mechanical plaque control: An overview //Indian Journal of Dental Sciences. - 2017. - T. 9. - № . 2. - C. 133.

24. Marchant R., Banat I. M. Biosurfactants: a sustainable replacement for chemical surfactants? //Biotechnology letters. - 2012. - T. 34. - № . 9. - C. 1597-1605.

25. Mnif I., Ghribi D. Glycolipid biosurfactants: main properties and potential applications in agriculture and food industry //Journal of the Science of Food and Agriculture. - 2016. - T. 96. - № . 13. - C. 4310-4320.

26. Moussa T. A. A., Mohamed M. S., Samak N. Production and characterization of dirhamnolipid produced by Pseudomonas aeruginosa TMN //Brazilian Journal of Chemical Engineering. - 2014. - T. 31. - C. 867-880.

27. Müller M. M. et al. Rhamnolipids-next generation surfactants? //Journal of biotechnology. - 2012. - T. 162. - № . 4. - C. 366-380.

28. Mynenivenkatasatya S. R. et al. Effectiveness of a novel dentifrice containing stabilized chlorine dioxide, sarkosyl, and sodium fluoride //Dentistry journal. - 2020. - T. 8. - № . 4. - C. 122.

29. Naughton P. J. et al. Microbial biosurfactants: current trends and applications in agricultural and biomedical industries //Journal of applied microbiology. - 2019. - T. 127. - № . 1. - C. 12-28.

30. Peyrat L. A. et al. Terrestrial microorganisms: cell factories of bioactive molecules with skin protecting applications //Molecules. - 2019. - T. 24. - № . 9. - C. 1836.

31. Santos D. K. F. et al. Biosurfactants: multifunctional biomolecules of the 21st century //International journal of molecular sciences. - 2016. - T. 17. - № . 3. - C. 401.

32. Sharma D. Biosurfactants in food. - Cham, Switzerland : Springer International Publishing, 2016.

33. Tackie-Otoo B. N. et al. Experimental investigation of N-lauroyl sarcosine and N-lauroyl-L-glutamic acid as green surfactants for enhanced oil recovery application //Journal of Molecular Liquids. - 2022. - T. 362. - C. 119738.

34. Tripathy D. B. et al. Synthesis, chemistry, physicochemical properties and industrial applications of amino acid surfactants: A review //Comptes Rendus Chimie. - 2018. - T. 21. - № . 2. - C. 112-130.

35. Vecino X. et al. Biosurfactants in cosmetic formulations: trends and challenges //Critical reviews in biotechnology. - 2017. - T. 37. - № . 7. - C. 911-923.

36. Vyas T. et al. Chemical plaque control-A brief review //Journal of Family Medicine and Primary Care. - 2021. - T. 10. - № . 4. - C. 1562.

37. Yamasaki R. et al. Rhamnolipids and surfactin inhibit the growth or formation of oral bacterial biofilm //BMC microbiology. - 2020. - T. 20. - № . 1. - C. 1-11.

## Claims

1. A composition comprising at least the following two components (A) and (B):

   (A) rhamnolipid; and
   (B) sodium lauroyl sarcosinate.

2. The composition of claim 1, wherein said rhamnolipid is derived from bacterium of the genus Burkholderia, preferably from Pseudomonas spp., further preferably from Pseudomonas aeruginosa, further preferably rhamnolipid having the CAS number 4348-76-9.

3. The composition of any of the preceding claims, wherein said composition comprises component (A) as per the following wt% ranges in said composition:

> i) 0.004 - 5.000, 0.006 - 4.000; 0.008 - 3.000; 0.010 - 2.000; 0.011 - 1.900; 0.012 - 1.800; 0.013 - 1.700; 0.014 - 1.600; 0.015 - 1.500; 0.015 - 1.400; 0.015 - 1.300; 0.015 - 1.200; 0.015 - 1.100; 0.015 - 1.000; or
> ii) 0.06-0.20; 0.07-0.19; 0.08-0.18; 0.09-0.17; 0.1-0.16; 0.11-0.15; 0.12-0.14; 0.13-0.14; or
> iii) any combination of an upper limit with an lower limited of the forementioned ranges.

4. The composition of any of the preceding claims, wherein said composition comprises component (B) as per the following wt% ranges in said composition:

> i) 0.05 - 22.00; 0.10 - 11.00; 0.20 - 5.50; or
> ii) 1.0-2.0; 1.1-1.9; 1.2-1.8; 1.3-1.7; 1.4-1.6; or 1.5-1.6; or
> iii) any combination of an upper limit with a lower limited of the forementioned ranges.

5. The composition of claim 1 or claim 2, wherein said the weight ratio of sodium lauroyl sarcosinate and rhamnolipid in said combination is 0.07:2.0 to 0.004:0.2.

6. The composition of any of the preceding claims, wherein said composition is an oral care composition, wherein said oral care composition is preferably selected from the group consisting of a mouthwash, toothpaste.

7. The composition of claim 6, wherein said oral care composition is a formulation selected from the following: film, aerosol, suspension, solution, tincture, cream, paste, lotion, ointment, gel, powder, granulate.

8. Pharmaceutical preparation comprising the composition of any of the preceding claims.

9. The composition of any of claims 1-7 for use as a medicament, wherein said medicament is preferably formulated for application in the mouth of a subject.

10. The pharmaceutical preparation of claim 8 or the composition for use of claim 9, wherein said pharmaceutical preparation or composition is for use

> - in the prophylaxis or treatment of an oral disease, wherein said oral disease is preferably selected from the group consisting of caries, periodontitis, gingivitis, tonsillitis, stomatitis, halitosis, dental calculus, or
> - in the treatment of an endodontic infection of the dental pulp, root or nerves of a tooth in a subject, wherein said tooth is preferably a living natural tooth.

11. The composition of any of claims 1-7, wherein said composition is for non-medical and/or aesthetic use.

12. Use of the composition of any of claims 1-7 in the treatment of plaque and/or biofilm or for prevention of a plaque and/or biofilm formation in a mouth and/or on teeth.

13. The use of claim 12, wherein said plaque and/or biofilm is derived from oral microorganisms in the mouth, wherein said oral microorganisms are preferably selected from the group consisting of *Streptococcus mutans, Streptococcus oralis, Streptococcus sanguinis, Neisseria mucosa* and *Actinomyces naeslundii.*

14. The use of claim 12 or claim 13, wherein said plaque and/or biofilm is on a solid surface of a tooth, preferably a supragingival plaque or a subgingival plaque.

15. Use of the composition of any of claims 1-7 in the treatment of plaque and/or biofilm or for prevention of a plaque and/or biofilm formation on an intraoral brace or prosthesis of a subject, including dentures, partial dentures, palatal obturators, dental implants, crowns, bridges, said treatment preferably *ex-vivo* or outside said subject.

**Fig. 1**

Fig. 2

Europäisches
Patentamt

European
Patent Office

Office européen
des brevets

# EUROPEAN SEARCH REPORT

Application Number

EP 23 17 3010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2019/307657 A1 (WENK HANS HENNING [DE] ET AL) 10 October 2019 (2019-10-10) * pages 14, 15, paragraph [0142] * * page 17, paragraph [0154] * * page 21, paragraph [0181] * * page 2, paragraph [0036] * * page 1, paragraph [0018] * ----- | 1-8, 10-15 | INV. A61K8/44 A61K8/60 A61Q11/00 A61Q11/02 A61K31/197 A61K31/7028 A61P1/02 |
| X | EP 3 875 100 A1 (PHYTOPLENUS BIOATIVOS S A [BR]) 8 September 2021 (2021-09-08) * pages 9, 10, paragraph [0066] * * page 20; claims 18-21 * ----- | 1-4,8,9, 11 | |
| A,D | US 2022/023180 A1 (WU SHUYUN [CN] ET AL) 27 January 2022 (2022-01-27) * page 1, paragraphs [0001], [0005] * ----- | 1-15 | |
| A,D | WO 2019/133313 A1 (LOCUS IP CO LLC [US]) 4 July 2019 (2019-07-04) * pages 25, 26; claims 1-20 * ----- | 1-15 | |
| | | | TECHNICAL FIELDS SEARCHED (IPC) A61K A61Q A61P |

The present search report has been drawn up for all claims

| Place of search | Date of completion of the search | Examiner |
|---|---|---|
| The Hague | 12 October 2023 | Opravz, Petra |

EPO FORM 1503 03.82 (P04C01)

## ANNEX TO THE EUROPEAN SEARCH REPORT
## ON EUROPEAN PATENT APPLICATION NO.

EP 23 17 3010

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2023

| Patent document cited in search report | | | Publication date | Patent family member(s) | | | Publication date |
|---|---|---|---|---|---|---|---|
| US | 2019307657 | A1 | 10-10-2019 | CN | 110267641 | A | 20-09-2019 |
| | | | | EP | 3579819 | A1 | 18-12-2019 |
| | | | | JP | 7271431 | B2 | 11-05-2023 |
| | | | | JP | 2020506214 | A | 27-02-2020 |
| | | | | US | 2019307657 | A1 | 10-10-2019 |
| | | | | WO | 2018145966 | A1 | 16-08-2018 |
| EP | 3875100 | A1 | 08-09-2021 | AU | 2019373411 | A1 | 20-05-2021 |
| | | | | BR | 102018072258 | A2 | 26-05-2020 |
| | | | | CA | 3117494 | A1 | 07-05-2020 |
| | | | | CN | 112996525 | A | 18-06-2021 |
| | | | | EP | 3875100 | A1 | 08-09-2021 |
| | | | | IL | 282629 | A | 30-06-2021 |
| | | | | JP | 2022514120 | A | 09-02-2022 |
| | | | | KR | 20210086670 | A | 08-07-2021 |
| | | | | US | 2021353699 | A1 | 18-11-2021 |
| | | | | WO | 2020087146 | A1 | 07-05-2020 |
| | | | | ZA | 202103074 | B | 26-10-2022 |
| US | 2022023180 | A1 | 27-01-2022 | CN | 113967173 | A | 25-01-2022 |
| | | | | EP | 4167941 | A1 | 26-04-2023 |
| | | | | US | 2022023180 | A1 | 27-01-2022 |
| | | | | WO | 2022020698 | A1 | 27-01-2022 |
| WO | 2019133313 | A1 | 04-07-2019 | CA | 3085343 | A1 | 04-07-2019 |
| | | | | EP | 3731804 | A1 | 04-11-2020 |
| | | | | US | 2020345610 | A1 | 05-11-2020 |
| | | | | WO | 2019133313 | A1 | 04-07-2019 |

EPO FORM P0459

For more details about this annex : see Official Journal of the European Patent Office, No. 12/82

## REFERENCES CITED IN THE DESCRIPTION

### Patent documents cited in the description

- US 2019307657 A1
- WO 2019133313 A1
- US 2021169757 A1
- US 7985722 B2
- US 2022023180 A1

### Non-patent literature cited in the description

- *CHEMICAL ABSTRACTS,* 4348-76-9
- **ASPINALL S. R. ; PARKER J. K. ; KHUTORYAN-SKIY V. V.** Oral care product formulations, properties and challenges. *Colloids and Surfaces B: Biointerfaces,* 2021, vol. 200, 111567
- **BAJANI D. ; GHARAI D. ; DEY J.** A comparison of the self-assembly behaviour of sodium N-lauroyl sarcosinate and sodium N-lauroyl glycinate surfactants in aqueous and aqueo-organic media. *Journal of colloid and interface science,* 2018, vol. 529, 314-324
- **FRACCHIA L. et al.** Industrial applications of biosurfactants. *Biosurfactants: production and utilization-processes, technologies, and economics.,* 2014, 245-260
- Future Prospects and Scenario of Rhamnolipids. **KUMAR R. ; DAS A. J.** Rhamnolipid Biosurfactant. Springer, 2018, 137-141
- Industrial Applications of Rhamnolipid: An Innovative Green Technology for Industry. **KUMAR R. ; DAS A. J.** Rhamnolipid Biosurfactant. Springer, 2018, 65-77
- **LANIGAN R. S.** Final report on the safety assessment of Cocoyl Sarcosine, Lauroyl Sarcosine, Myristoyl Sarcosine, Oleoyl Sarcosine, Stearoyl Sarcosine, Sodium Cocoyl Sarcosinate, Sodium Lauroyl Sarcosinate, Sodium Myristoyl Sarcosinate, Ammonium Cocoyl Sarcosinate, and Ammonium Lauroyl Sarcosinate. *International journal of toxicology,* 2001, vol. 20, 1-14
- **LINDENMÜLLER I. H. ; LAMBRECHT J. T.** Oral care. *Topical applications and the mucosa.,* 2011, vol. 40, 107-115
- **MOUSSA T. A. A. ; MOHAMED M. S. ; SAMAK N.** Production and characterization of dirhamnolipid produced by Pseudomonas aeruginosa TMN. *Brazilian Journal of Chemical Engineering,* 2014, vol. 31, 867-880
- **SHARMA D.** Biosurfactants in food. Springer International Publishing, 2016
- **TACKIE-OTOO B. N. et al.** Experimental investigation of N-lauroyl sarcosine and N-lauroyl-L-glutamic acid as green surfactants for enhanced oil recovery application. *Journal of Molecular Liquids,* 2022, vol. 362, 119738